# EUROPEAN PATENT APPLICATION

(11) **EP 4 710 871 A1**
(43) Date of publication of application: **18.03.2026**
(21) Application number: 24822610.2
(22) Date of filing: 05.06.2024
(51) Int. Cl.: A61B 17/04, A61B 17/062

(54) **NEEDLE DRIVING ASSEMBLY UNDER ENDOSCOPE, AND SUTURING DEVICE**

(30) Priority: 12.06.2023 CN 202310696267
(71) Applicant: Zhang, Qiang, Guangzhou, Guangdong 510515 (CN); Micro-Tech (Nanjing) Co., Ltd., Nanjing, Jiangsu 210032 (CN)
(72) Inventor: ZHANG, Qiang, Guangzhou, Guangdong 510515 (CN)
(74) Representative: Rondano, Davide
(86) International application number: PCT/CN2024/097486
(87) International publication number: WO 2024/255659

(57) **Abstract**

The present application provides a needle driving assembly under an endoscope, and a suturing device. The needle driving assembly under an endoscope comprises: a housing assembly, an arc-shaped suture needle, a rotating body, a driving assembly, a control member, a snap-fitting member and a limiting member. The control member cooperates the driving assembly to control the rotating body to rotate, and the rotating body drives the snap-fitting member to rotate in a first direction or a second direction, so that the arc-shaped suturing needle rotates by one circle relative to the housing assembly, so as to thread human tissue with a needle one time. The structure has a rational design, is compact, and is convenient to use, and by means of cooperation of the rotating body, the driving assembly, the control member, the snap-fitting member and the limiting member, the arc-shaped suturing needle is driven to rotate by 360 degrees repeatedly relative to the housing assembly, so as to achieve the suturing of the human tissue, thereby improving operation efficiency. In addition, the movement direction of the control member is at least partially perpendicular to the rotation direction of the rotating body and the arc-shaped suturing needle, thereby conveniently observing the suture state of the arc-shaped suturing needle and adapting to the suture operation under a flexible or rigid endoscope.

## Description

### TECHNICAL FIELD

The present invention relates to the field of endoscopic minimally invasive technology, and in particular to an endoscopic needle driving assembly and a suturing device.

### BACKGROUND ART

With the development of medical technology, endoscopic minimally invasive surgery is applied to treat more and more diseases. Endoscopic minimally invasive surgery is to implement surgery through a natural cavity of the human body or forms a small entry path from a body surface of the human body to enter the human body. The surgery has small trauma, good treatment effect, and fast postoperative recovery. In a minimally invasive surgery process, suturing is the most common problem. Usually, a good suturing instrument needs to satisfy characteristics of high efficiency and safety. At present, in minimally invasive surgery under a surgical endoscope, a C-type suturing instrument deserves attention. The suturing instrument realizes suturing through driving a C-type arc needle to perform needle-penetrating and thread-guiding. The suturing instrument implements suturing in a space required for rotation of the arc needle. Since an operation space required by the suturing instrument is small, therefore, there is little limit about the size of the operation space. The arc needle performs circular rotation in a visual range of an operator to perform suturing, and the process is very safe. At present, the C-type suturing instrument under the surgical endoscope generally realizes suturing through transmission structures such as connecting rods and double wheels; since technology of the suturing instrument is protected, the price of the suturing instrument is high, popularization and promotion are limited, making it difficult to let more patients benefit. Breaking existing technical barriers and letting more patients in need use the type instrument is worthy to explore. In addition, different from the surgical endoscope, a method for suturing a wound surface under a flexible endoscope in minimally invasive surgery is relatively lacking of diversity, and it is mainly by a metal endoscopic clip. The method has lower strength and reliability in suturing the wound surface compared with surgical thread suturing, so its application is limited by wound surface size and full-thickness suturing. The above C-type suturing instrument has a very big advantage in operation. Therefore, developing a C-type suturing instrument under the flexible endoscope is also worthy doing. At present, there are very few C-type suturing instruments available for use through flexible endoscope. The prior art is still at a very early development stage, in which the reliability of the suturing outcome remains uncertain, the structure is complex, too many components are involved and the reliability is low.

### SUMMARY

To achieve the above objectives, the present invention provides an endoscopic needle driving assembly comprising: a housing assembly, wherein the housing assembly comprises a suturing groove and an arc-shaped suturing hole arranged along a circumferential direction of the suturing groove, and the suturing groove is configured to accommodate human tissue; an arc-shaped suturing needle, wherein the arc-shaped suturing needle is arranged in the arc-shaped suturing hole in a rotatable manner; a rotating body, wherein the rotating body is mounted in the housing assembly in a rotatable manner; a driving assembly, wherein the driving assembly is arranged in the housing assembly and is connected with the rotating body; a control member, wherein the control member is connected with the driving assembly, a rotation axis of the rotating body is parallel to an extension direction of at least a part of the control member, and the control member are configured to cooperate with the driving assembly to drive the rotating body to rotate; a snap-fit member, wherein the snap-fit member is connected with the rotating body and is able to engage the arc-shaped suturing needle; and a limiting member, wherein the limiting member is arranged in the housing assembly and is able to prevent the arc-shaped suturing needle from retracting; when the rotating body drives the snap-fit member to rotate along a first direction relative to the housing assembly, the snap-fit member is able to engage the arc-shaped suturing needle, and the limiting member slides relative to the arc-shaped suturing needle; when the rotating body drives the snap-fit member to rotate along a second direction relative to the housing assembly, the snap-fit member is able to be separated from the arc-shaped suturing needle, and the limiting member is able to prevent the arc-shaped suturing needle from retracting; and the first direction is opposite to the second direction.

In the above endoscopic needle driving assembly, the rotating body is provided with engaging teeth, and the driving assembly cooperates with the engaging teeth.

In the above endoscopic needle driving assembly, the driving assembly comprises a first gear and a first pulley; the first gear is fixedly connected with the first pulley and is coaxial with the first pulley; the first gear cooperates with the engaging teeth; the first pulley is provided with a first annular groove and a second annular groove, wherein one end of the control member is fixed in the first annular groove, and the other end of the control member is fixed in the second annular groove and is wound in the second annular groove.

In the above endoscopic needle driving assembly, the driving assembly comprises two second gears cooperating with the engaging teeth, and an annular groove is provided on each of the two second gears; one end of the control member is fixed in the annular groove of one of the two second gears, and the other end of the control member is fixed and wound in the annular groove of the other one of the two second gears.

In the above endoscopic needle driving assembly, the driving assembly comprises a pulley, and the control member is redirected by the pulley and then connected to the rotating body.

In the above endoscopic needle driving assembly, the rotating body is a bevel gear.

In the above endoscopic needle driving assembly, the rotating body is the second pulley, and the second pulley is provided with a third annular groove and a fourth annular groove; two pulleys are provided; one end of the control member is fixed in the third annular groove after being redirected by one pulley; and the other end of the control member is fixed in the fourth annular groove after being redirected by another pulley, and is wound in the fourth annular groove.

In the above endoscopic needle driving assembly, the rotating body is provided with an arc-shaped sliding groove; the arc-shaped sliding groove is wound around the rotating body; and in an axial direction of the rotating body, the arc-shaped sliding groove extends from one end of the rotating body to the other end of the rotating body; the driving assembly comprises a sliding block, wherein the sliding block is connected with the control member and is slidably mounted in the arc-shaped sliding groove.

In the above endoscopic needle driving assembly, the driving assembly further comprises a fixing shell, wherein the fixing shell is fixed in the housing assembly, and along a length direction of the fixing shell, the fixing shell is provided with a sliding hole; a portion of the sliding block is located in the fixing shell and is slidable in the fixing shell, and another portion of the sliding block passes through the sliding hole and is inserted into the arc-shaped sliding groove; and a spring, wherein the spring is arranged in the fixing shell and is compressed in place between the sliding block and the fixing shell.

In the above endoscopic needle driving assembly, the housing assembly comprises: a mounting shell; and a fixing tube, wherein the fixing tube is fixed in the mounting shell, and the rotating body is sleeved over the fixing tube and is able to rotate relative to the fixing tube.

In the above endoscopic needle driving assembly, the fixing tube comprises a fixing section and a mounting section, a diameter of the mounting section is smaller than a diameter of the fixing section, and the rotating body is fixed on the mounting section.

In the above endoscopic needle driving assembly, the fixing tube comprises a tube body and a transfer shaft, the rotating body is able to be mounted on the transfer shaft in a rotatable manner, and a projection of the rotating body on the tube body at most covers a part of the through hole of the tube body.

In the above endoscopic needle driving assembly, a side wall of the suturing groove is provided with a cut surrounding the suturing groove, and the cut communicates with the suturing hole.

In the above endoscopic needle driving assembly, the rotating body is coaxial with the arc-shaped suturing needle.

In the above endoscopic needle driving assembly, a rotation axis of the rotating body has an angle with a rotation axis of the arc-shaped suturing needle.

Compared with existing technologies, the above technical solution has the following advantages.

An operator controls rotation of the rotating body through cooperation of the control member and the driving assembly, and the rotating body drives the snap-fit member to rotate along a first direction or a second direction relative to the housing assembly. When controlling the snap-fit member to move from an initial position along the first direction, the snap-fit member engages the arc-shaped suturing needle, and the limiting member slides relative to the arc-shaped suturing needle, causing the snap-fit member to drive the arc-shaped suturing needle to rotate 180° relative to the housing assembly, to make the arc-shaped suturing needle penetrate human tissue. When the snap-fit member reaches an end position of movement, the snap-fit member is controlled to move along the second direction, the limiting member prevents the arc-shaped suturing needle from retracting, and the snap-fit member is separated from the arc-shaped suturing needle, so as to make the snap-fit member return to the initial position. By repeating the above operations, the arc-shaped suturing needle is enabled to rotate one revolution relative to the housing assembly, thereby making the arc-shaped suturing needle penetrate human tissue. By repeating the process, the arc-shaped suturing needle rotates multiple times relative to the housing assembly, thereby completing suturing of human tissue. The above structure is reasonably designed, compact, and easy to use. Through cooperation between the rotating body, the driving assembly, the control member, the snap-fit member, and the limiting member, the arc-shaped suturing needle is driven to rotate repeatedly multiple times 360° relative to the housing assembly, thereby suturing human tissue and improving surgical operation efficiency. In addition, a movement direction of the control member is at least partially perpendicular to rotation directions of the rotating body and the arc-shaped suturing needle, thus facilitating observation of a suturing state of the arc-shaped suturing needle and adapting to suturing operations under flexible or rigid endoscopes. In addition, the rotating body itself and its cooperation with main structures have unique spatial positions and cooperation relationships. For example, a rotation axis of the rotating body and the arc-shaped suturing needle is at least partially parallel to a pushing or pulling direction of the control member. Based on the above features, the technical solution can be applied not only under a surgical endoscope but also installed on a flexible endoscope, providing a broader application scenario. In addition, the technical solution has a simple and compact structural design, it does not involve complex miniature precision components, and it reduces manufacturing processes.

The embodiment of a suturing device of a second aspect of the present invention comprises a suture, an endoscope and the endoscopic needle driving assembly described above. The suture is connected with the arc-shaped suturing needle of the endoscopic needle driving assembly. The endoscope is sleeved and connected with the fixing tube of the endoscopic needle driving assembly.

Compared with existing technologies, the above technical solution has the following advantages.

The technical solution is provided with implementation effect features of an existing C-shaped suturing instrument and also is provided with unique advantages. The technical solution is mainly implemented through cooperation among the rotating body, the driving assembly, the control member, the snap-fit member and the limiting member. Different from C-shaped suturing instruments under surgical endoscopes in the prior art, the rotating body itself and its cooperation with main structures provide unique spatial positions and cooperation relationships. For example, a rotation axis of the rotating body and the arc-shaped suturing needle is at least partially parallel to a pushing or pulling direction of the control member. Based on the above features, the technical solution can be applied not only under a surgical endoscope but also installed on a flexible endoscope, providing a broader application scenario. In addition, the technical solution has a simple and compact structural design, does not involve complex miniature precision components, and reduces manufacturing processes.

### BRIEF DESCRIPTION OF DRAWINGS

The following figures are provided only for illustrative purposes to explain the present invention and do not limit the scope of the present invention.
FIG. 1 is a structural and basic principle diagram of an endoscopic needle driving assembly according to the present invention;
FIG. 2 is a structural schematic diagram of a first embodiment of an endoscopic needle driving assembly of the present invention;
FIG. 3 is a sectional schematic diagram of the endoscopic needle driving assembly shown in FIG. 2;
FIG. 4a is an exploded structural schematic diagram of the endoscopic needle driving assembly shown in FIG. 2;
FIG. 4b is an enlarged schematic diagram of region A in FIG. 4a;
FIG. 5 is a structural schematic diagram of an endoscopic needle driving assembly shown in FIG. 2 in a first state when suturing human tissue;
FIG. 6 is a partial sectional view of a snap-fit member in a first state in FIG. 5;
FIG. 7 is a structural schematic diagram of an endoscopic needle driving assembly shown in FIG. 2 in a second state when suturing human tissue;
FIG. 8 is a partial sectional view of a snap-fit member in a second state in FIG. 7;
FIG. 9 is a structural schematic diagram of a snap-fit member in a third state when an endoscopic needle driving assembly shown in FIG. 2 sutures human tissue;
FIG. 10 is a structural schematic diagram of a snap-fit member in a fourth state when an endoscopic needle driving assembly shown in FIG. 2 sutures human tissue;
FIG. 11 is a structural schematic diagram of a snap-fit member in a fifth state when an endoscopic needle driving assembly shown in FIG. 2 sutures human tissue;
FIG. 12 is a structural schematic diagram of a cooperation structure of a snap-fit member and a rotating body shown in FIG. 2;
FIG. 13 is a structural schematic diagram of a driving assembly shown in FIG. 2;
FIG. 14 is a sectional schematic diagram of a second embodiment of an endoscopic needle driving assembly (bevel gear transmission) of the present invention;
FIG. 15 is a sectional schematic diagram of a third embodiment of an endoscopic needle driving assembly of the present invention;
FIG. 16 is a structural schematic diagram of a fourth embodiment of an endoscopic needle driving assembly of the present invention;
FIG. 17 is a sectional schematic diagram of the endoscopic needle driving assembly shown in FIG. 16;
FIG. 18 is an exploded structural schematic diagram of the endoscopic needle driving assembly shown in FIG. 16;
FIG. 19 is a structural schematic diagram of a driving assembly shown in FIG. 16;
FIG. 20 is a structural schematic diagram of a fifth embodiment of an endoscopic needle driving assembly of the present invention;
FIG. 21 is a sectional schematic diagram of the endoscopic needle driving assembly shown in FIG. 20;
FIG. 22 is an exploded structural schematic diagram of the endoscopic needle driving assembly shown in FIG. 20;
FIG. 23 is a structural schematic diagram of a sixth embodiment of an endoscopic needle driving assembly of the present invention;
FIG. 24 is a sectional schematic diagram of the endoscopic needle driving assembly shown in FIG. 23;
FIG. 25 is an exploded structural schematic diagram of the endoscopic needle driving assembly shown in FIG. 23.

Reference numerals
10, housing assembly; 11, mounting shell; 111, body; 112, top cover; 12, fixing tube; 121, fixing section; 122, mounting section; 123, tube body; 124, transfer shaft; 13, arc-shaped suturing hole; 14, cut; 15, relief notch; 16, suturing groove; 17, relief groove;
20, arc-shaped suturing needle; 21, connecting hole; 22, snap-fit plate; 23, snapping groove;
30, rotating body; 31, engaging teeth; 32, second pulley; 33, arc-shaped sliding groove;
40, driving assembly; 41, first gear; 42, first pulley; 421, first annular groove; 422, second annular groove; 43, second gear; 431, annular groove; 44, pulley; 45, sliding block; 46, fixing shell; 47, spring;
50, control member;
60, snap-fit member; 61, connecting shell; 62, limiting rod; 63, supporting spring;
70, limiting member; 71, first piece; 72, second piece;
80, fixing ring;
100, endoscopic needle driving assembly;
200, suture;
300, endoscope.

### DETAILED DESCRIPTION OF EMBODIMENTS

The present invention will be further described in detail below based on the drawings and embodiments. Through the descriptions, characteristics and advantages of the present invention will become clearer and more definite.

The term "for example" as used here means "used as an example, embodiment, or illustrative". Any embodiment described as "for example" herein should not be construed as being superior to or better than other embodiments. Although various aspects of the embodiments are shown in the drawings, the drawings need not be to scale unless specifically indicated.

Moreover, the technical features involved in different embodiments of the present invention described below may be combined with each other as long as they do not constitute conflicts. The following discussion is provided with multiple embodiments of the present invention. Although each embodiment represents a single combination of the present invention, different embodiments of the present invention can be replaced or merged for combination. Therefore, the present invention can also be regarded as including all possible combinations of same and/or different embodiments described. Thus, if one embodiment contains A, B, C, another embodiment contains a combination of B and D, then the present invention also should be regarded as including one or more embodiments of all other possible combinations containing A, B, C, D, although the embodiment may not have explicit written description in the following content. Moreover, the technical features involved in different embodiments of the present invention described below may be combined with each other as long as they do not constitute conflicts.

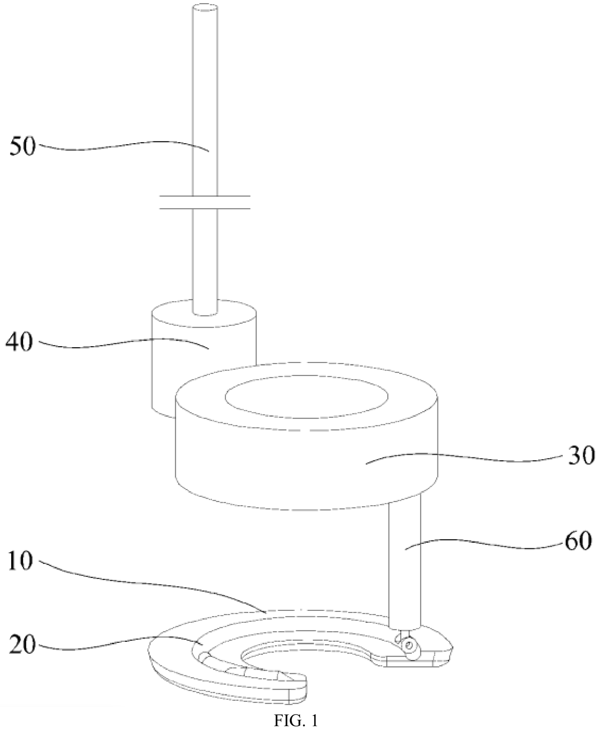

As shown in FIG. 1, the main structure of an endoscopic needle driving assembly 100 includes: a housing assembly 10, an arc-shaped suturing needle 20, a rotating body 30, a driving assembly 40, a control member 50, and a snap-fit member 60. The schematic diagram shows a basic principle method implemented by the technical solution. The technical solution can be used for a C-type suturing instrument under a surgical endoscope rigid scope. Then the endoscopic needle driving assembly is connected with a rigid short cannula with an operation handle, becoming an instrument that can be independently implemented; at the same time, the endoscopic needle driving assembly also can be mounted on a long flexible endoscope to perform suturing operation.

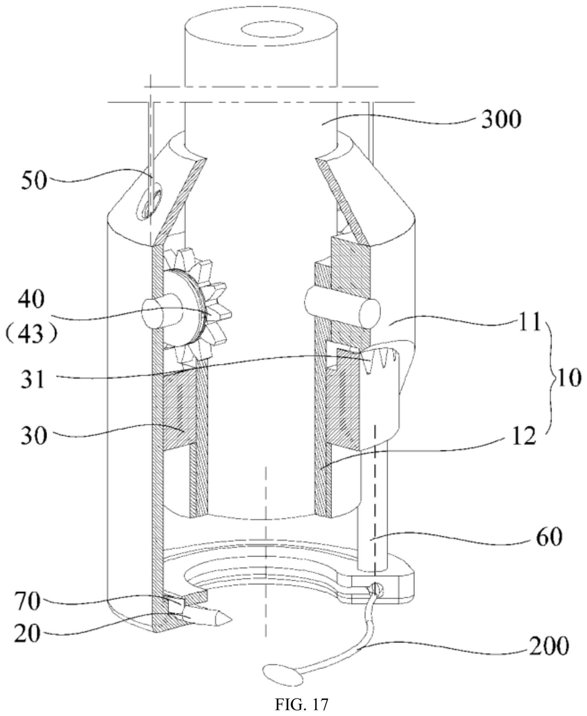

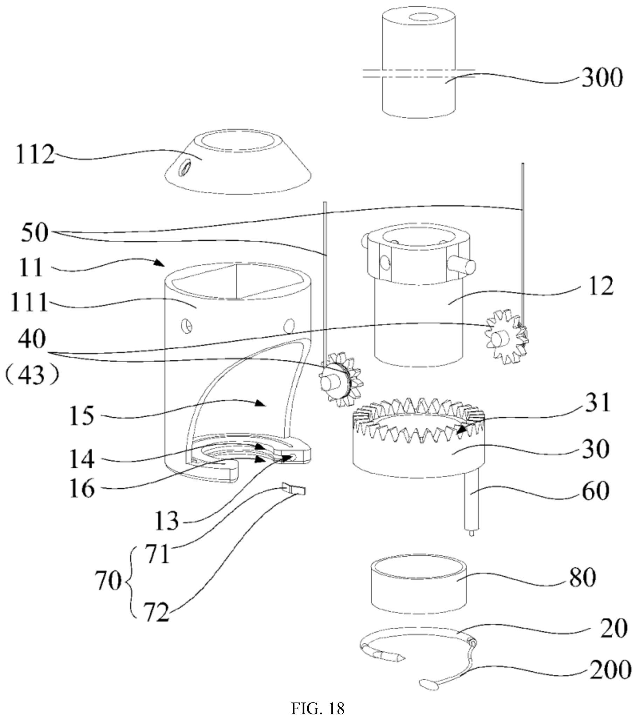

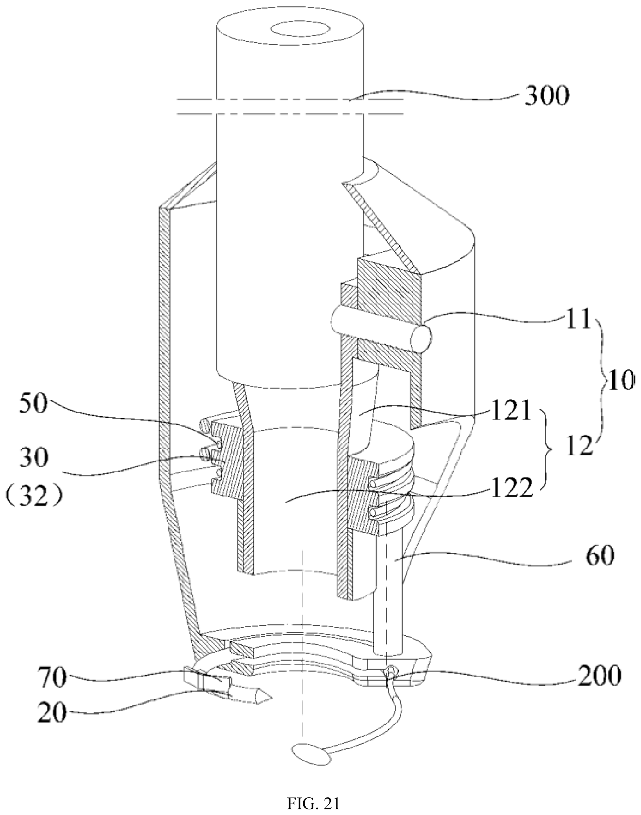

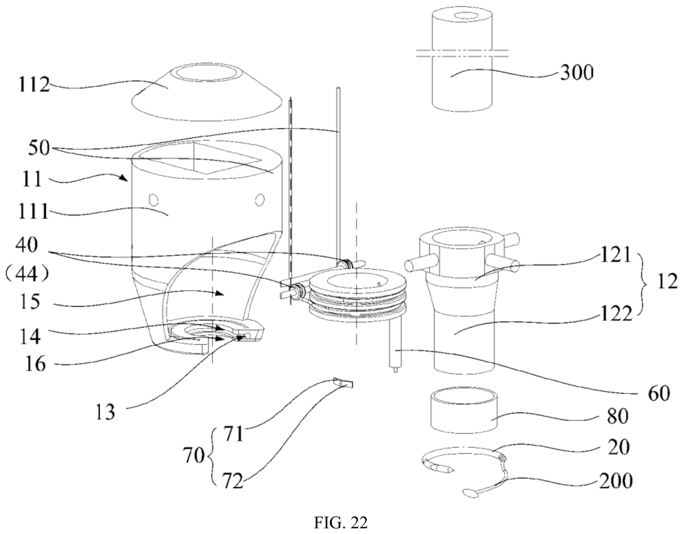

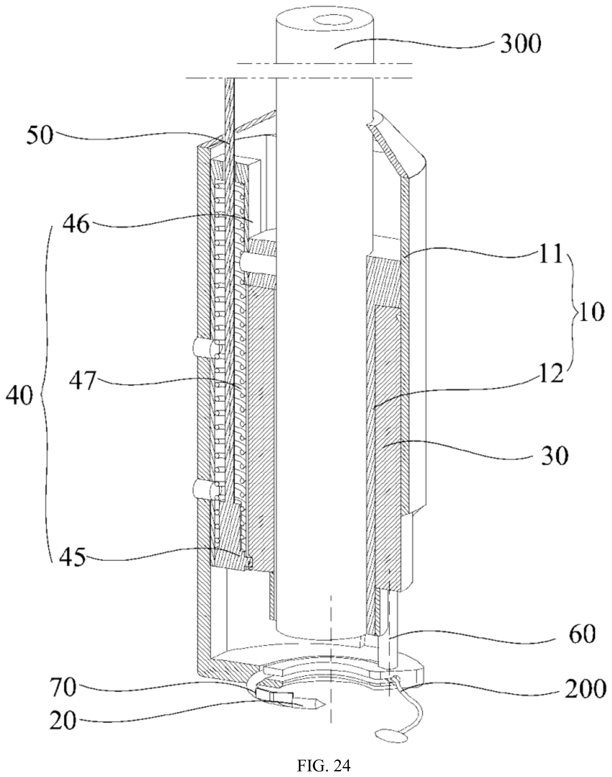

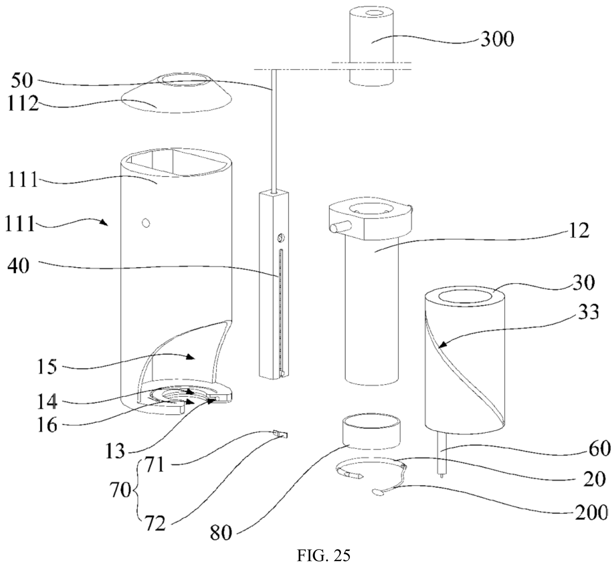

As shown in FIG. 1, FIG. 2 to FIG. 4b, FIG. 16 to FIG. 20, FIG. 20 to FIG. 24, FIG. 23 to FIG. 25, an embodiment of a first aspect of the present invention provides an endoscopic needle driving assembly 100 including the housing assembly 10, the arc-shaped suturing needle 20, the rotating body 30, the driving assembly 40, the control member 50, the snap-fit member 60, and a limiting member 70.

The housing assembly 10 includes a suturing groove 16 and an arc-shaped suturing hole 13 arranged along a circumferential direction of the suturing groove 16, and the suturing groove 16 is configured to accommodate human tissue. In a specific embodiment of the present invention, the housing assembly 10 includes: a mounting shell 11 and a fixing tube 12.

The mounting shell 11 has an accommodating cavity, the mounting shell 11 includes a top plate and a bottom plate that are oppositely arranged, and a side surrounding plate arranged between the top plate and the bottom plate. The top plate is provided with a mounting hole for an endoscope 300 to pass through, the bottom plate is provided with the suturing groove 16, and the arc-shaped suturing hole 13 is arranged along the circumferential direction of the suturing groove 16. The side surrounding plate is provided with a relief notch 15 communicated with the suturing groove 16, and the suturing groove 16 is configured to accommodate human tissue. In one embodiment of the present invention, the mounting shell can be a split structure, the mounting shell 11 includes a body 111 and a top cover 112.

The fixing tube 12 is fixed in the mounting shell 11, the rotating body 30 is sleeved over the fixing tube 12 and can rotate relative to the fixing tube 12. The fixing tube 12 can be sleeved over a front end of the endoscope 300.

The arc-shaped suturing needle 20 is arranged in the arc-shaped suturing hole 13 in a rotatable manner.

The rotating body 30 is mounted in the housing assembly 10 in a rotatable manner. In a specific embodiment of the present invention, the rotating body 30 is fixed on the fixing tube 12 through a fixing ring 80.

The driving assembly 40 is arranged in the housing assembly 10 and is connected with the rotating body 30.

The control member 50 is connected with the driving assembly 40, a rotation axis of the rotating body 30 is parallel to an extension direction of at least a part of the control member 50, and the control member 50 are configured to cooperate with the driving assembly 40 to drive the rotating body 30 to rotate. The control member 50 can be flexible or can be rigid, and a person skilled in the art can select the corresponding control member 50 according to specific requirements.

The snap-fit member 60 is connected with the rotating body 30 and can engage the arc-shaped suturing needle 20.

The limiting member 70 is arranged in the housing assembly 10 and can prevent the arc-shaped suturing needle 20 from retracting.

When the rotating body 30 drives the snap-fit member 60 to rotate along a first direction relative to the housing assembly 10, the snap-fit member 60 can engage the arc-shaped suturing needle 20, and the limiting member 70 slides relative to the arc-shaped suturing needle 20. When the rotating body 30 drives the snap-fit member 60 to rotate along a second direction relative to the housing assembly 10, the snap-fit member 60 can be separated from the arc-shaped suturing needle 20, and the limiting member 70 can prevent the arc-shaped suturing needle 20 from retracting. The first direction is opposite to the second direction.

By the endoscopic needle driving assembly 100 provided in the present invention, an operator controls rotation of the rotating body 30 through cooperation of the control member 50 and the driving assembly 40, and the rotating body 30 drives the snap-fit member 60 to rotate along a first direction or a second direction relative to the housing assembly 10. When controlling the snap-fit member 60 to move from an initial position along the first direction, the snap-fit member 60 engages the arc-shaped suturing needle 20, and the limiting member 70 slides relative to the arc-shaped suturing needle 20, causing the snap-fit member 60 to drive the arc-shaped suturing needle 20 to rotate 180° relative to the housing assembly 10, to make the arc-shaped suturing needle 20 penetrate human tissue. When the snap-fit member 60 reaches an end position of movement, the snap-fit member 60 is controlled to move along the second direction, the limiting member 70 prevents the arc-shaped suturing needle 20 from retracting, and the snap-fit member 60 separates from the arc-shaped suturing needle 20, so as to make the snap-fit member 60 return to the initial position. By repeating the above operation, the arc-shaped suturing needle 20 rotates one turn relative to the housing assembly 10, to make the arc-shaped suturing needle 20 penetrate human tissue. By analogy, the arc-shaped suturing needle 20 rotates multiple turns relative to the housing assembly 10 to complete suturing of human tissue. The above structure design is simple and reasonable. The structure is compact, without many miniature precision components, thus reducing processing difficulty. Through cooperation between the rotating body 30, the driving assembly 40, the control member 50, the snap-fit member 60, and the limiting member 70, the arc-shaped suturing needle 20 is driven to repeatedly rotate 360° relative to the housing assembly 10 to achieve suturing of human tissue. The operation is simple, which is not limited by narrow operation space. During rotation of the suturing needle, no surrounding human tissue and organs are damaged, thereby greatly improving surgery efficiency and safety.

In addition, since a rotation axis of the rotating body 30 and a rotation axis of the arc-shaped suturing needle 20 are at least partially and substantially parallel to a longitudinal axis of a distal end of a long flexible endoscope, the endoscopic needle driving assembly 100 does not completely block a working channel outlet and light source at a front end of the endoscope. This allows the operator to advance other endoscopic accessories through the working channel and to perform surgical maneuvers while continuously observing the arc-shaped suturing needle(20 and the tissue being sutured in real time.

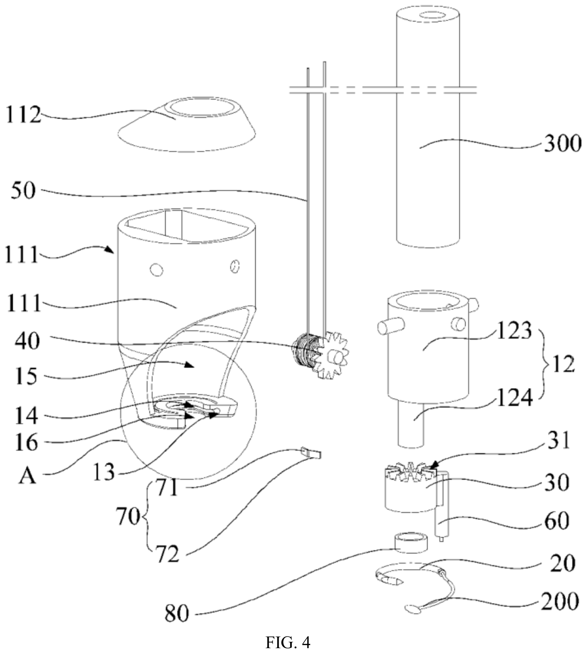

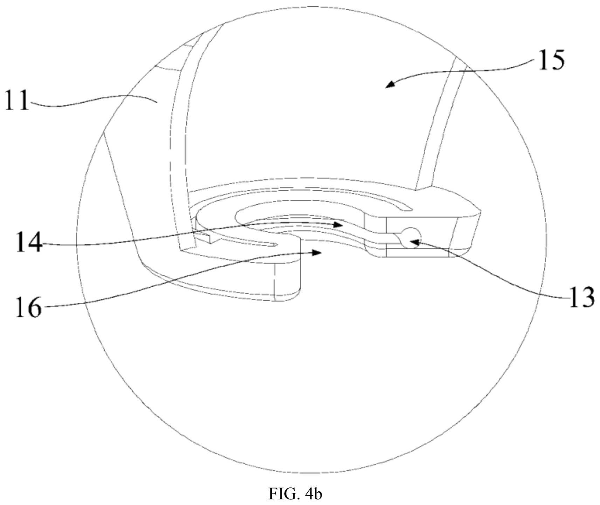

As shown in FIG. 4a, FIG. 4b, FIG. 18, FIG. 22, and FIG. 25, in an embodiment of the present invention, a side wall of the suturing groove 16 is provided with a cut 14 surrounding the suturing groove 16, and the cut 14 communicates with the suturing hole.

After the arc-shaped suturing needle 20 rotates one turn under the action of the rotating body 30, the driving assembly 40, the control member 50, the snap-fit member 60, and the limiting member 70, with rotation of the arc-shaped suturing needle 20, a suture 200 connected with the arc-shaped suturing needle 20 also penetrates human tissue. During this process, the suture 200 slides out of the arc-shaped suturing hole 13 from the cut 14, which avoids occurrence of the suture 200 winding on the housing assembly 10.

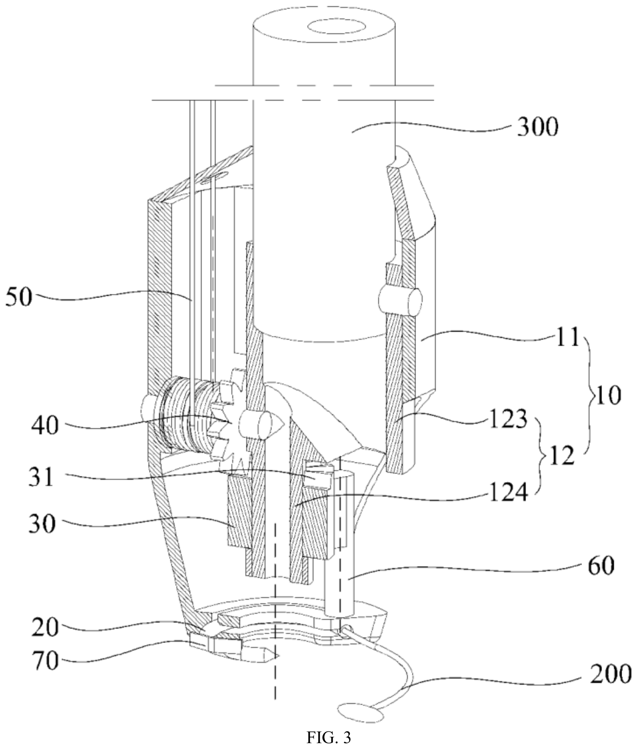

As shown in FIG. 3, FIG. 14, FIG. 21, and FIG. 24, in an embodiment of the present invention, the rotating body 30 is coaxial with the arc-shaped suturing needle 20.

The structure enables the rotating body 30 and the arc-shaped suturing needle 20 to have the same rotation angle. By controlling the rotation angle of the rotating body 30, the rotation angle of the arc-shaped suturing needle 20 can be controlled, so that rotation of the rotating body 30 is precisely transmitted to the arc-shaped suturing needle 20, causing the arc-shaped suturing needle 20 to rotate along a standard circular path. This improves the suturing accuracy and reliability.

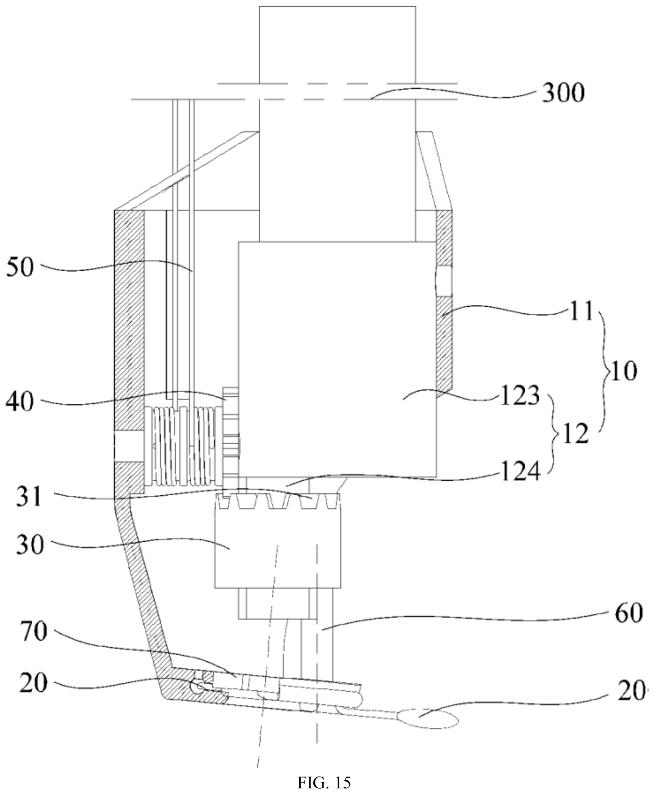

As shown in FIG. 15, in an embodiment of the present invention, there is an angle between the rotation axis of the rotating body 30 and the rotation axis of the arc-shaped suturing needle 20.

The structure makes the arc-shaped suturing needle 20 inclined at an angle relative to the rotating body 30, so that the suturing groove 16 matches a visual field and a working channel of the endoscope. During the suturing of human tissue, the arc-shaped suturing needle 20 is easy to pass out from the arc-shaped suturing hole 13 and accurately penetrate human tissue. At the same time, the operator observes the arc-shaped suturing needle and suturing state of human tissue in real time through the endoscope. In the embodiment, the rotating body 30 and the arc-shaped suturing needle 20 are projected coaxially on a plane parallel to the rotating body 30; and the rotating body 30 and the arc-shaped suturing needle 20 have the same rotation angle to precisely drive suturing needle rotation.

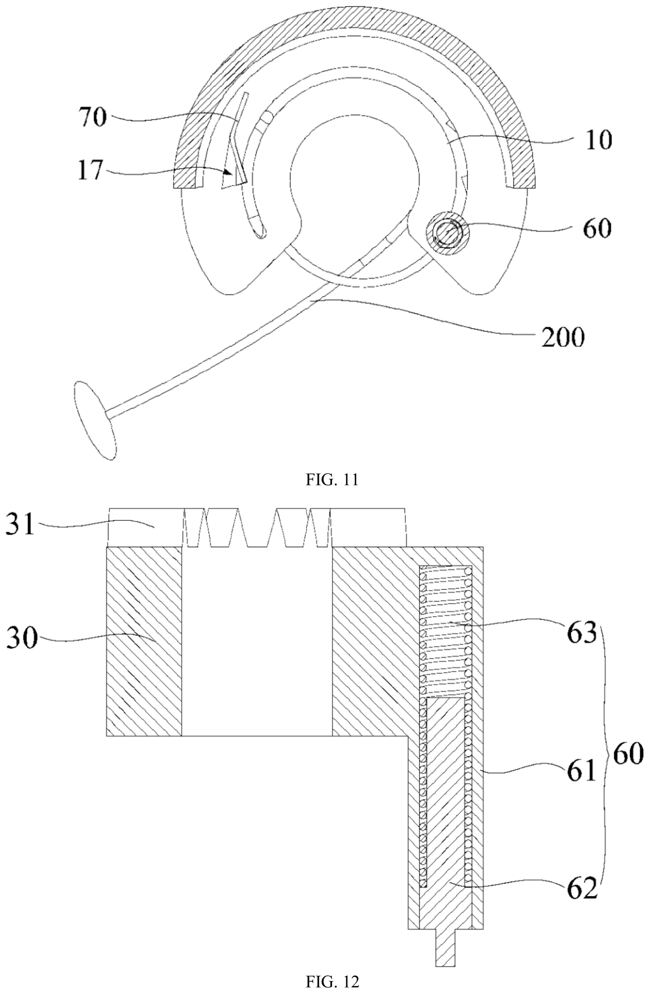

As shown in FIG. 12, in an embodiment of the present invention, each snap-fit member 60 includes: a connecting shell 61, a limiting rod 62, and a supporting spring 63.

The connecting shell 61 is fixed on the rotating body 30.

One end of the limiting rod 62 inserts into the connecting shell 61, the other end of the limiting rod 62 protrudes from the connecting shell 61 and can engage a snap-fit structure on the arc-shaped suturing needle 20.

The supporting spring 63 is compressed in place between the limiting rod 62 and the connecting shell 61. Specifically, the limiting rod 62 is provided with a supporting platform along its circumferential direction, the supporting spring 63 is sleeved over the limiting rod 62 and compressed in place between the supporting platform and the connecting shell 61.

When controlling the snap-fit member 60 to move from the initial position along the first direction, under action of the supporting spring 63, the other end of the limiting rod 62 protrudes from the connecting shell 61 and engages the snapping face of a snap-fit structure, thereby enabling the snap-fit member 60 to drive the arc-shaped suturing needle 20 to rotate and move a distance relative to the housing assembly 10. When controlling the snap-fit member 60 to move along the second direction, the limiting rod 62 compresses the supporting spring 63 along a guide surface of the snap-fit structure, so that the snap-fit member 60 separates from the snap-fit structure, enabling the snap-fit member 60 to return to the initial position. The snap-fit member 60 have a simple structure, and is easy to manufacture, thereby reducing production cost of the product.

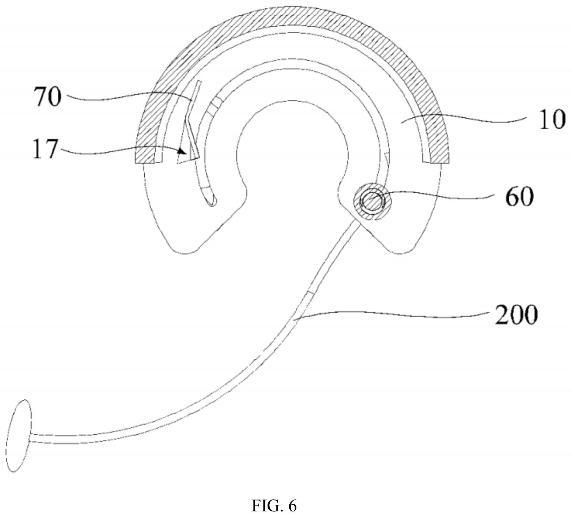

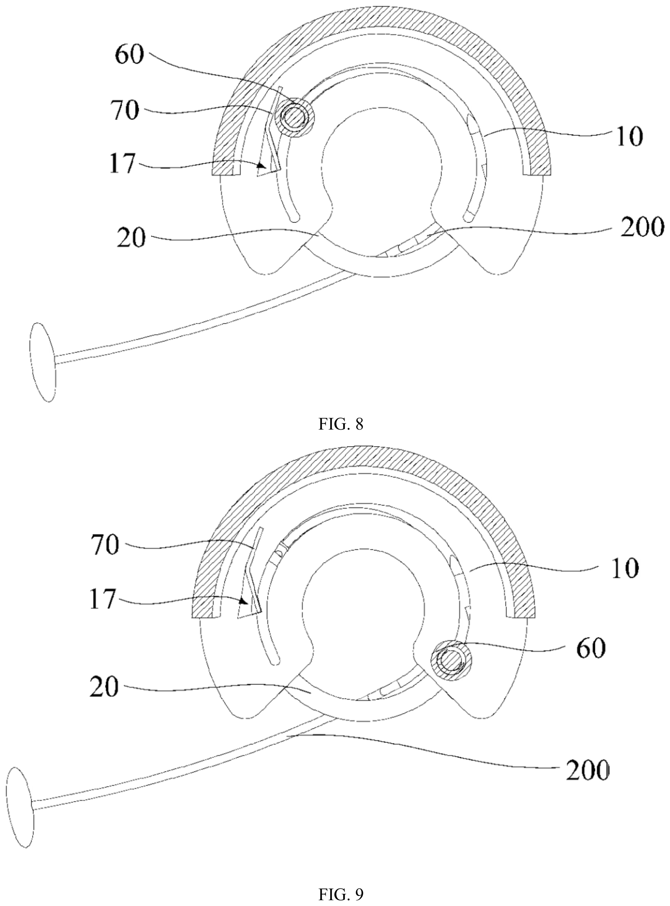

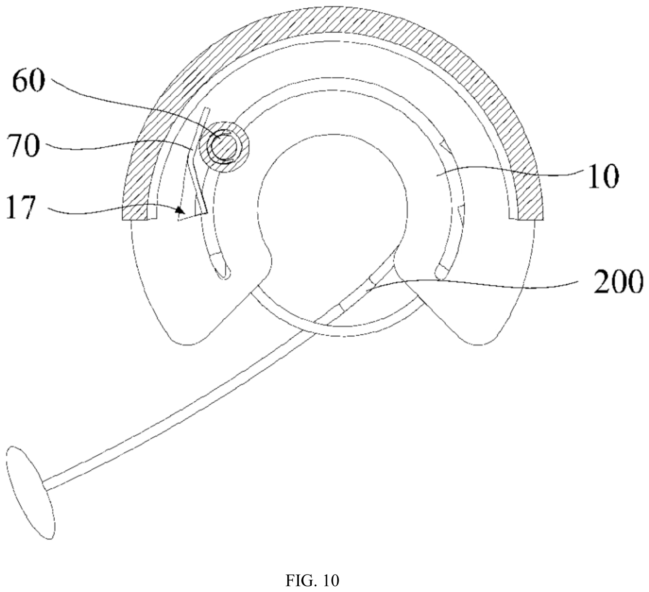

As shown in FIG. 4a, FIG. 18, FIG. 22, and FIG. 25, in an embodiment of the present invention, the limiting member 70 is an elastic piece, the elastic piece includes a first piece 71 and a second piece 72, wherein the first piece 71 and the second piece 72 are connected. The first piece 71 is connected with the housing assembly 10, and the second piece 72 presses on the arc-shaped suturing needle 20. In addition, as shown in FIG. 6, FIG. 8 to FIG. 11, the housing assembly 10 also is provided with a relief groove 17, and the relief groove 17 is provided with space for deformation of the elastic piece.

When controlling the snap-fit member 60 to move along the second direction, the elastic piece presses on a limiting surface of the limiting structure, preventing the arc-shaped suturing needle 20 from retracting. When controlling the snap-fit member 60 to move along the first direction, the elastic piece moves along an inclined surface of the limiting structure, causing the elastic piece to deform, thereby making the elastic piece slide relative to the arc-shaped suturing needle 20.

In a specific embodiment of the present invention, an outer surface at one end of the side surrounding plate connected with the bottom plate is provided with a first inclined surface.

When the endoscopic needle driving assembly enters a narrow space, the first inclined surface is provided with guiding function, allowing the endoscopic needle driving assembly to enter the narrow space more smoothly, thereby improving product usage safety and convenience.

In a specific embodiment of the present invention, an outer surface of the top plate is provided with a second inclined surface, causing the top plate to have a frustoconical shape.

The structure allows the top end of the mounting shell to be rounded, avoiding the mounting shell scratching human tissue, thus ensuring product usage safety.

The following specifically describes several embodiments of the endoscopic needle-driving assembly in combination with the drawings.

### Embodiment 1

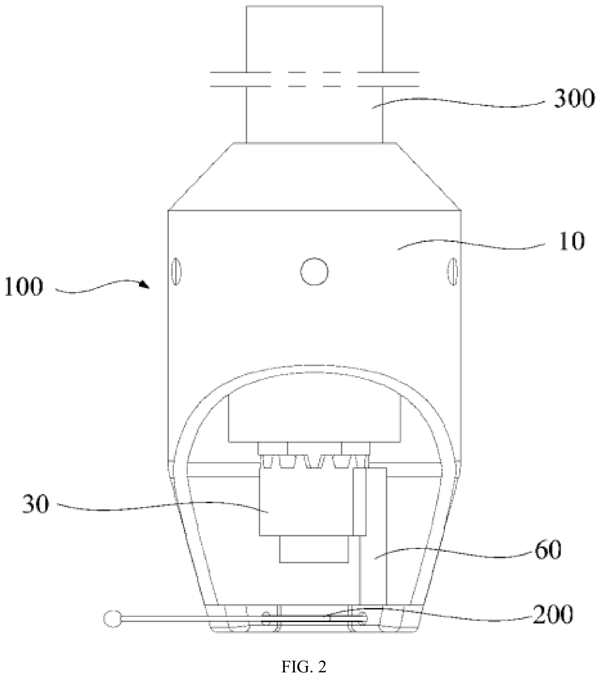

As shown in FIG. 2 to FIG. 4b, the endoscopic needle-driving assembly 100 includes: the housing assembly 10, the arc-shaped suturing needle 20, the rotating body 30, the driving assembly 40, the control member 50, the snap-fit member 60, and the limiting member 70. Specifically, the control member 50 is a cable.

The housing assembly 10 includes: a mounting shell 11 and a fixing tube 12.

The fixing tube 12 is fixed in the mounting shell 11, the rotating body 30 is sleeved over the fixing tube 12 and can rotate relative to the fixing tube 12.

The fixing tube 12 includes a tube body 123 and a transfer shaft 124, the rotating body 30 can be mounted on the transfer shaft 124 in a rotatable manner, a projection of the rotating body 30 on the tube body 123 at most covers a part of the through hole of the tube body 123. Specifically, the transfer shaft 124 is located on a side of the tube body 123 away from the suturing groove 16. The structure avoids the situation where the rotating body 30 sleeved over the transfer shaft 124 completely blocks the entire through hole, allowing the endoscope 300 to observe the arc-shaped suturing needle 20 and suturing state of human tissue through the through hole, and making endoscopic surgical accessories extend from a working channel of the endoscope 300. In addition, the rotating body 30 is fixed on the transfer shaft 124 rather than sleeved over a peripheral side of a distal end of the endoscope 300, so that an overall outer diameter of the endoscopic needle-driving assembly 100 can be smaller, thereby reducing an operation space required for the endoscopic needle-driving assembly 100, and making operation more convenient.

The rotating body 30 is provided with engaging teeth 31, the driving assembly 40 cooperates with the engaging teeth 31.

The driving assembly 40 includes a first gear 41 and a first pulley 42; the first gear 41 is fixedly connected with the first pulley 42, and is coaxial with the first pulley 42; and the first gear 41 cooperates with the engaging teeth 31.

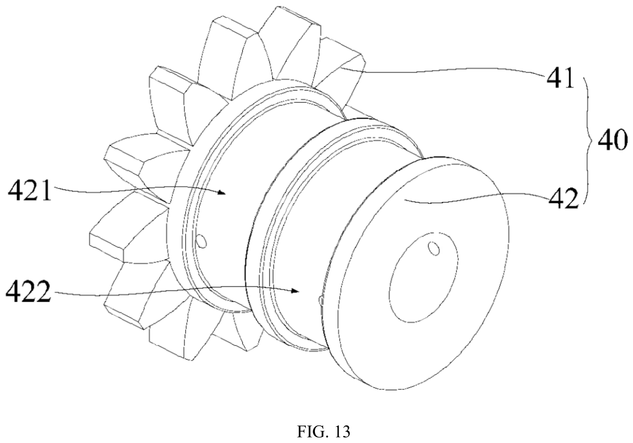

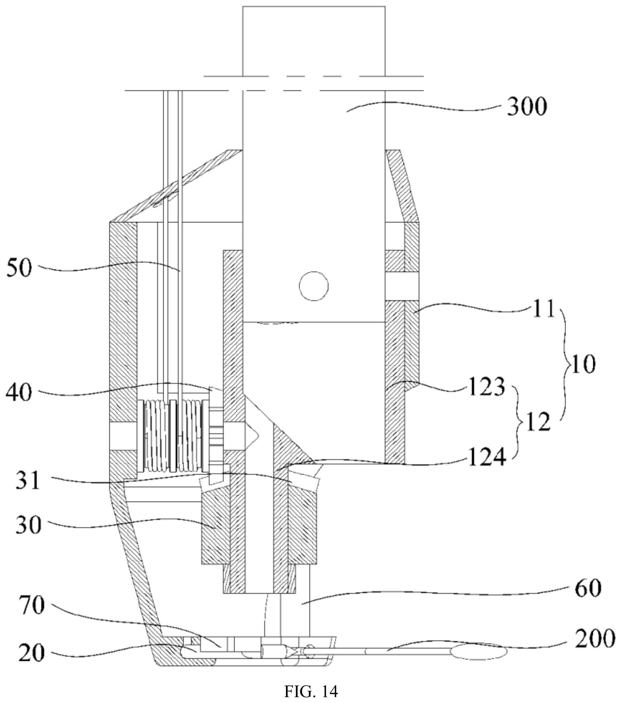

As shown in FIG. 13, the first pulley 42 is provided with a first annular groove 421 and a second annular groove 422, where one end of the control member 50 is fixed in the first annular groove 421, and the other end of the control member 50 is fixed in the second annular groove 422 and wound in the second annular groove 422. As shown in FIG. 14, in a specific embodiment of the present invention, the rotating body 30 is a bevel gear, that is, the rotating body 30 and the first gear 41 cooperate in bevel gear transmission.

The structure uses a gear transmission mode, which has long service life, smooth operation, and high reliability, thereby ensuring effective driving of the snap-fit member 60, improving product usage reliability.

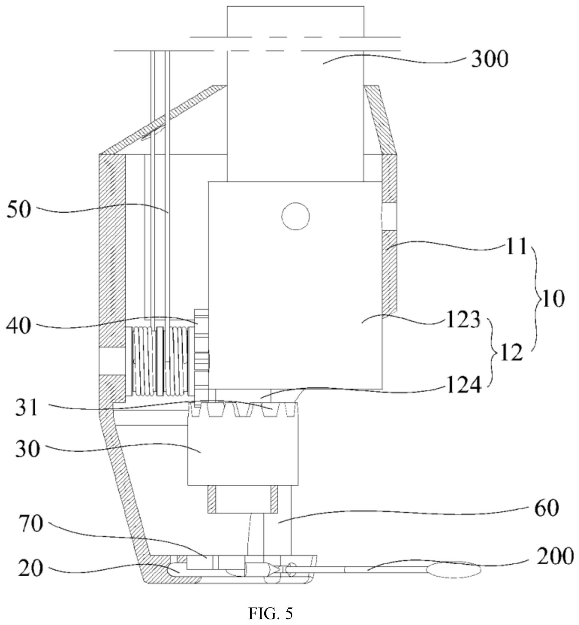

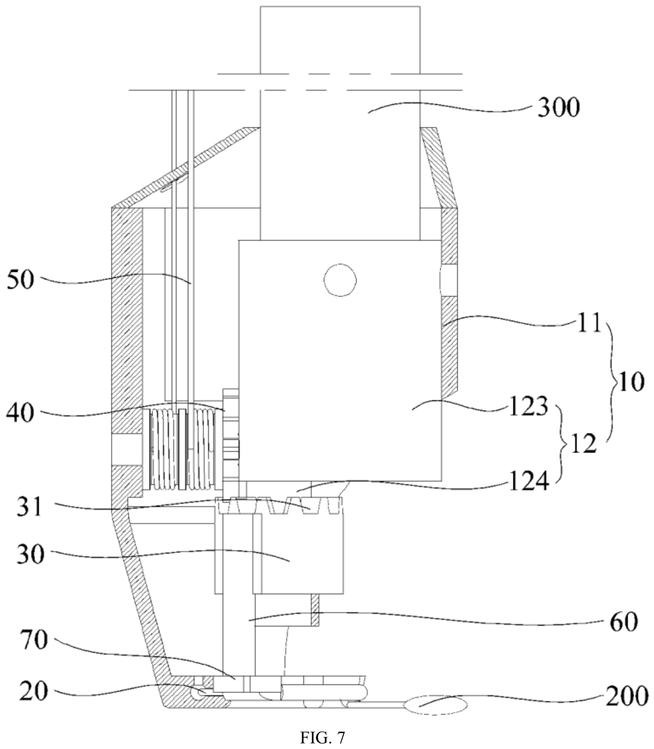

As shown in FIG. 5 to FIG. 11, the working process of the endoscopic needle-driving assembly 100 is as follows.

The operator pulls the control member 50 in a forward direction. One end of the control member 50 is wound in the first annular groove 421, and the other end of the control member 50 is partially wound in the second annular groove 422. This action causes the first pulley 42 to rotate, thereby driving the first gear 41 to rotate. The first gear 41 drives the rotating body 30 to rotate. The rotating body 30 drives the snap-fit member 60 to rotate from the initial position along the first direction. The snap-fit member 60 engages the arc-shaped suturing needle 20, and the limiting member 70 slides relative to the arc-shaped suturing needle 20, causing the snap-fit member 60 to drive the arc-shaped suturing needle 20 to rotate 180° relative to the housing assembly 10, thereby making the arc-shaped suturing needle 20 penetrate human tissue. When the snap-fit member 60 reaches the end position of movement, the operator pulls the control member 50 in a reverse direction. One end of the control member 50 is partially wound in the first annular groove 421, and the other end of the control member 50 is wound in the second annular groove 422. This action causes the first pulley 42 to rotate, driving the first gear 41 to rotate. The first gear 41 drives the rotating body 30 to rotate. The rotating body 30 drives the snap-fit member 60 to move along the second direction. The limiting member 70 prevents the arc-shaped suturing needle 20 from retracting, and the snap-fit member 60 separates from the arc-shaped suturing needle 20, so that the snap-fit member 60 returns to the initial position. By repeating the above operations, the arc-shaped suturing needle 20 is enabled to rotate 360° relative to the housing assembly 10, thereby making the arc-shaped suturing needle 20 penetrate human tissue. By repeating the process, the arc-shaped suturing needle 20 rotates 360° multiple times relative to the housing assembly 10, thereby completing suturing of human tissue.

### Embodiment 2

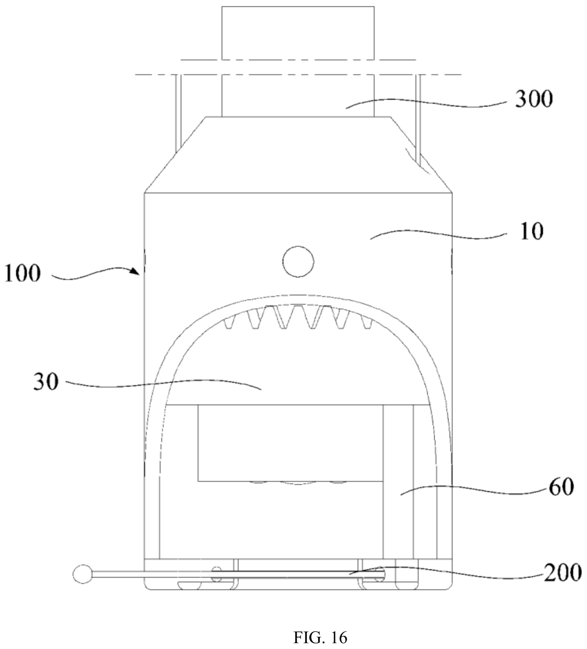

As shown in FIG. 16 to FIG. 19, the endoscopic needle-driving assembly 100 includes: the housing assembly 10, the arc-shaped suturing needle 20, the rotating body 30, the driving assembly 40, the control member 50, the snap-fit member 60, and the limiting member 70. Specifically, the control member 50 is a cable.

The housing assembly 10 includes: a mounting shell 11 and a fixing tube 12.

The fixing tube 12 is fixed in the mounting shell 11, the rotating body 30 is sleeved over the fixing tube 12 and can rotate relative to the fixing tube 12. The front end of the fixing tube 12 is unobstructed, and the rotating body 30 is located outside the field of view. Therefore, the field of view of the endoscope 300 is enlarged, so that the arc-shaped suturing needle 20 and the suturing state of human tissue can be more effectively observed through the endoscope 300 via the through hole of the fixing tube 12. Moreover, a larger operation space can be provided after endoscopic surgical accessories extend from the working channel of the endoscope 300.

The rotating body 30 is provided with engaging teeth 31, the driving assembly 40 cooperates with the engaging teeth 31.

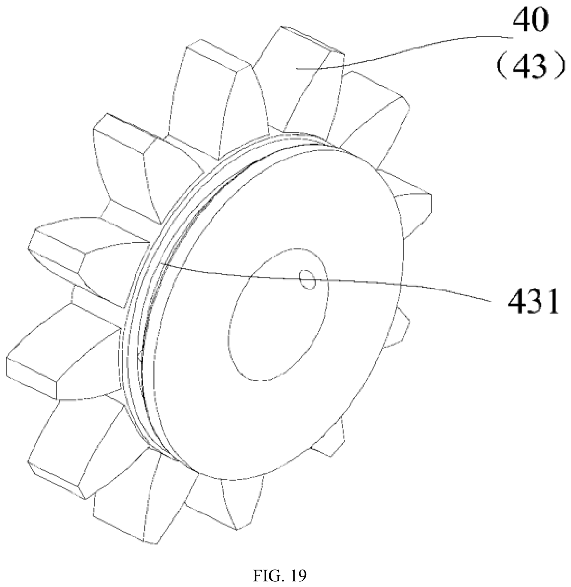

In a specific embodiment of the present invention, the driving assembly 40 includes two second gears 43 cooperating with the engaging teeth 31, as shown in FIG. 19. An annular groove 431 is provided on each of the two second gears 43. Specifically, the two second gears 43 are symmetrically arranged on both sides of the fixing tube 12.

One end of the control member 50 is fixed in the annular groove 431 of one second gear 43, and the other end of the control member 50 is fixed in the annular groove 431 of the other second gear 43 and wound in the annular groove 431.

The structure uses a gear transmission mode, which has long service life, smooth operation, and high reliability, thereby ensuring effective driving of the snap-fit member 60, improving product usage reliability.

The working process of the endoscopic needle-driving assembly 100 is as follows.

The operator pulls the control member 50 in a forward direction. The other end of the control member 50 is partially wound in the annular groove 431 of the other second gear 43, causing the second gear 43 to rotate. Another second gear 43 drives the rotating body 30 to rotate, and the rotating body 30 drives the snap-fit member 60 to move from an initial position along the first direction. The snap-fit member 60 engages the arc-shaped suturing needle 20, and the limiting member 70 slides relative to the arc-shaped suturing needle 20, causing the snap-fit member 60 to drive the arc-shaped suturing needle 20 to rotate 180° relative to the housing assembly 10, to make the arc-shaped suturing needle 20 penetrate human tissue. During this process, the rotating body 30 drives one second gear 43 to rotate, one end of the control member 50 is wound in the annular groove 431 of the one second gear 43. When the snap-fit member 60 reaches the end position of movement, the operator pulls the control member 50 in a reverse direction. One end of the control member 50 is partially wound in the annular groove 431 of the one second gear 43, causing the second gear 43 to rotate. The one second gear 43 drives the rotating body 30 to rotate. The rotating body 30 drives the snap-fit member 60 to move along the second direction. The limiting member 70 prevents the arc-shaped suturing needle 20 from retracting, and the snap-fit member 60 separates from the arc-shaped suturing needle 20, returning the snap-fit member 60 to the initial position. During this process, the rotating body 30 drives the other second gear 43 to rotate, and the other end of the control member 50 is wound in the annular groove 431 of the other second gear 43. By repeating the above operations, the arc-shaped suturing needle 20 is enabled to rotate 360° relative to the housing assembly 10, thereby making the arc-shaped suturing needle 20 penetrate human tissue. By repeating the process, the arc-shaped suturing needle 20 rotates 360° multiple times relative to the housing assembly 10, thereby completing suturing of human tissue.

### Embodiment 3

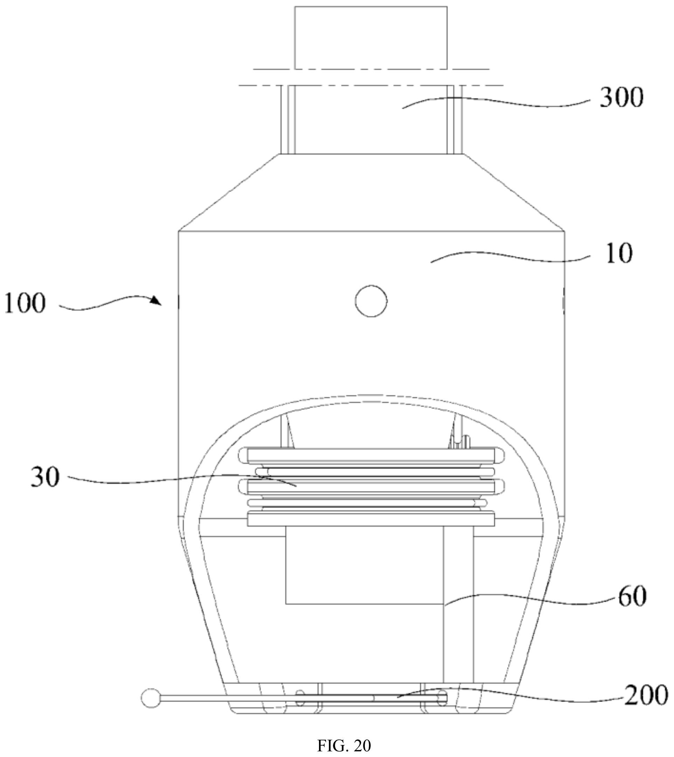

As shown in FIG. 20 to FIG. 22, the endoscopic needle-driving assembly 100 includes: the housing assembly 10, the arc-shaped suturing needle 20, the rotating body 30, the driving assembly 40, the control member 50, the snap-fit member 60, and the limiting member 70. Specifically, the control member 50 is a cable.

The housing assembly 10 includes: a mounting shell 11 and a fixing tube 12.

The fixing tube 12 is fixed in the mounting shell 11, the rotating body 30 is sleeved over the fixing tube 12 and can rotate relative to the fixing tube 12.

The fixing tube 12 includes a fixing section 121 and a mounting section 122, the diameter of the mounting section 122 is smaller than the diameter of the fixing section 121, and the rotating body 30 is fixed on the mounting section 122. The arc-shaped suturing needle 20 and the suturing state of human tissue can be observed through the through hole of the mounting section 122 by the endoscope 300. In addition, the rotating body 30 is fixed on the mounting section 122; this reduces the space occupied by the rotating body 30, and makes the overall volume of the endoscopic needle driving assembly 100 smaller.

The driving assembly 40 includes a pulley 44, the control member 50 is redirected by the pulley 44 and then connected to the rotating body 30. Specifically, two pulleys 44 are provided.

The rotating body 30 is the second pulley 32, and the second pulley 32 is provided with a third annular groove and a fourth annular groove.

One end of the control member 50 is fixed in the third annular groove after being redirected by one pulley 44; and the other end of the control member 50 is fixed in the fourth annular groove after being redirected by another pulley 44, and is wound in the fourth annular groove.

The structure above has a long service life, smooth operation, high reliability, and a simple configuration. This ensures effective driving of the snap-fit member 60, thereby improving product usage reliability.

The working process of the endoscopic needle-driving assembly 100 is as follows.

The operator pulls the control member 50 in a forward direction. One end of the control member 50 is wound in the third annular groove, and the other end of the control member 50 is partially wound in the fourth annular groove. This action causes the second pulley 32 to rotate, thereby driving the snap-fit member 60 to rotate from the initial position along the first direction. The snap-fit member 60 engages the arc-shaped suturing needle 20, and the limiting member 70 slides relative to the arc-shaped suturing needle 20, causing the snap-fit member 60 to drive the arc-shaped suturing needle 20 to rotate 180° relative to the housing assembly 10, thereby making the arc-shaped suturing needle 20 penetrate human tissue. When the snap-fit member 60 reaches the end position of movement, the operator pulls the control member 50 in a reverse direction. One end of the control member 50 is partially wound in the third annular groove, and the other end of the control member 50 is wound in the fourth annular groove. This action causes the second pulley 32 to rotate, driving the snap-fit member 60 to move along the second direction. The limiting member 70 prevents the arc-shaped suturing needle 20 from retracting, and the snap-fit member 60 separates from the arc-shaped suturing needle 20, so that the snap-fit member 60 returns to the initial position. By repeating the above operations, the arc-shaped suturing needle 20 is enabled to rotate 360° relative to the housing assembly 10, thereby making the arc-shaped suturing needle 20 penetrate human tissue. By repeating the process, the arc-shaped suturing needle 20 rotates 360° multiple times relative to the housing assembly 10, thereby completing suturing of human tissue.

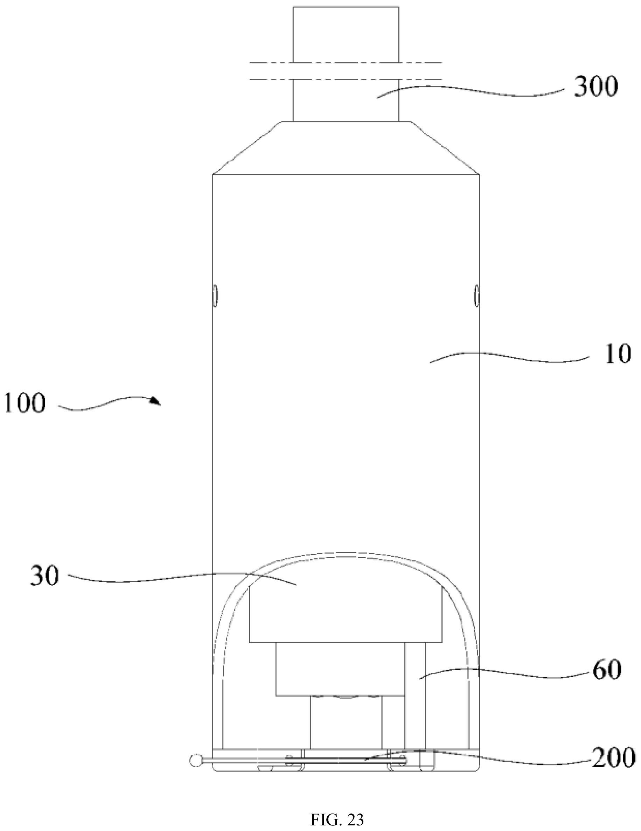

In further embodiment,
As shown in FIG. 23 to FIG. 25, the endoscopic needle-driving assembly 100 includes: the housing assembly 10, the arc-shaped suturing needle 20, the rotating body 30, the driving assembly 40, the control member 50, the snap-fit member 60, and the limiting member 70. Specifically, the control member 50 can be a pushing-pulling member with a hardness or a flexible cable.

The housing assembly 10 includes: a mounting shell 11 and a fixing tube 12.

The fixing tube 12 is fixed in the mounting shell 11, the rotating body 30 is sleeved over the fixing tube 12 and can rotate relative to the fixing tube 12. The front end of the fixing tube 12 is unobstructed, and the rotating body 30 is located outside the field of view. Therefore, the field of view of the endoscope 300 is relatively big, so that the arc-shaped suturing needle 20 and the suturing state of human tissue can be more effectively observed through the endoscope 300 via the through hole of the fixing tube 12. Moreover, a larger operation space can be provided after endoscopic surgical accessories extend from the working channel of the endoscope 300.

The rotating body 30 is provided with an arc-shaped sliding groove 33. The arc-shaped sliding groove 33 is wound around the rotating body 30. In an axial direction of the rotating body 30, the arc-shaped sliding groove 33 extends from one end of the rotating body 30 to the other end of the rotating body 30.

The driving assembly 40 includes a fixing shell 46, a sliding block 45 and a spring 47.

The fixing shell 46 is fixed in the housing assembly 10. Along a length direction of the fixing shell 46, the fixing shell 46 is provided with a sliding hole.

The sliding block 45 is connected with the control member 50 and is slidably mounted in the arc-shaped sliding groove 33. Specifically, a portion of the sliding block 45 is located in the fixing shell 46 and is slidable in the fixing shell 46. Another portion of the sliding block 45 passes through the sliding hole and is inserted into the arc-shaped sliding groove 33.

The spring 47 is arranged in the fixing shell 46 and is compressed in place between the sliding block 45 and the fixing shell 46.

The structure above has a long service life, smooth operation, and high reliability. This ensures effective driving of the snap-fit member 60, thereby improving product usage reliability.

The working process of the endoscopic needle-driving assembly 100 is as follows.

The operator pulls the control member 50. The control member 50 compresses the spring 47. At this moment, the spring 47 stores energy. The control member 50 drives the sliding block 45 to slide in the arc-shaped sliding groove 33 along a forward direction, causing the rotating body 30 to rotate, and thereby causing the snap-fit member 60 to rotate from an initial position along a first direction. The snap-fit member 60 engages the arc-shaped suturing needle 20 and the limiting member 70 slides relative to the arc-shaped suturing needle 20, causing the snap-fit member 60 to drive the arc-shaped suturing needle 20 to rotate 180° relative to the housing assembly 10. Therefore, the arc-shaped suturing needle 20 is enabled to penetrate human tissue. When the snap-fit member 60 reaches an end position of movement, the operator removes the pulling force applied to the control member 50. Under an elastic force of the spring 47 or by simultaneously pushing the control member 50, the control member 50 is driven to be restored. The control member 50 drives the sliding block 45 to slide in the arc-shaped sliding groove 33 along a reverse direction, causing the rotating body 30 to rotate, and thereby causing the snap-fit member 60 to move along a second direction. The limiting member 70 prevents the arc-shaped suturing needle 20 from retracting. The snap-fit member 60 separates from the arc-shaped suturing needle 20, returning the snap-fit member 60 to the initial position. By repeating the above operations, the arc-shaped suturing needle 20 is enabled to rotate relative to the housing assembly 10, thereby making the arc-shaped suturing needle 20 penetrate human tissue. By repeating the process, the arc-shaped suturing needle 20 rotates multiple times relative to the housing assembly 10, thereby completing suturing of human tissue.

As shown in FIGS. 2 to 4a, FIGS. 16 to 18, FIGS. 20 to 22, and FIGS. 23 to 25, an embodiment of a suturing device of a second aspect of the present invention includes a suture 200, an endoscope 300 and the endoscopic needle driving assembly 100 described above. The suture 200 is connected with the arc-shaped suturing needle 20 of the endoscopic needle driving assembly100. The endoscope 300 is sleeved and connected with the fixing tube 12 of the endoscopic needle driving assembly 100.

The suturing device provided by the present invention is provided with reasonable structural design, compact structure, simple operation, convenient use, and enables continuous suturing of human tissue, thus greatly reducing difficulty of surgical operation and improving market competitiveness of the product.

Based on a basic principle and method of the technical solution shown in FIG. 1, the present invention proposes four representative specific embodiments. It should be understood that, based on the principle method of this technology, various alternatives, modifications, variations or improvements can be implemented by those skilled in the art. These technical solutions are all intended to be included in the present invention. In particular, structures of the driving assembly and the rotating body and a cooperation relationship between the driving assembly and the rotating body, except for the four solutions proposed by the present invention, are all included in the present invention as long as they can be implemented in this field. In addition, based on the principle and method of this technology, the four specific solutions proposed by the present invention and other technical solutions that can be implemented by those skilled in the art are applicable to flexible endoscopic suturing instruments and also applicable to rigid endoscopic suturing instruments of surgical endoscopy. These technical solutions are all intended to be included in the present invention.

In the description of the present invention, it is necessary to state that the terms "first" and "second" serve only for description purposes and cannot be construed as indicating or implying relative importance.

In the description of the present invention, it is necessary to state that unless otherwise expressly specified and limited, the terms "mount", "connect" and "connect with" are to be construed broadly. The term "multiple" refers to two or more unless otherwise expressly limited. Those of ordinary skill in the art can understand the meanings of the above terms in the present invention according to specific situations.

The above describes the present invention in combination with preferred embodiments. These embodiments are merely exemplary and serve only illustrative purposes. Based on these, various substitutions and improvements can be made to the present invention, and all such modifications fall within a protection scope of the present invention.

## Claims

1. An endoscopic needle driving assembly, wherein
the endoscopic needle driving assembly comprises:
a housing assembly, wherein the housing assembly comprises a suturing groove and an arc-shaped suturing hole arranged along a circumferential direction of the suturing groove, and the suturing groove is configured to accommodate human tissue;
an arc-shaped suturing needle, wherein the arc-shaped suturing needle is arranged in the arc-shaped suturing hole in a rotatable manner;
a rotating body, wherein the rotating body is mounted in the housing assembly in a rotatable manner;
a driving assembly, wherein the driving assembly is arranged in the housing assembly and is connected with the rotating body;
a control member, wherein the control member is connected with the driving assembly, a rotation axis of the rotating body is parallel to an extension direction of at least a part of the control member, and the control member are configured to cooperate with the driving assembly to drive the rotating body to rotate;
a snap-fit member, wherein the snap-fit member is connected with the rotating body and is able to engage the arc-shaped suturing needle; and
a limiting member, wherein the limiting member is arranged in the housing assembly and is able to prevent the arc-shaped suturing needle from retracting;
when the rotating body drives the snap-fit member to rotate along a first direction relative to the housing assembly, the snap-fit member is able to engage the arc-shaped suturing needle, and the limiting member slides relative to the arc-shaped suturing needle; when the rotating body drives the snap-fit member to rotate along a second direction relative to the housing assembly, the snap-fit member is able to be separated from the arc-shaped suturing needle, and the limiting member is able to prevent the arc-shaped suturing needle from retracting; and the first direction is opposite to the second direction.

2. The endoscopic needle driving assembly according to claim 1, wherein
the rotating body is provided with engaging teeth, and the driving assembly cooperates with the engaging teeth.

3. The endoscopic needle driving assembly according to claim 2, wherein
the driving assembly comprises a first gear and a first pulley; the first gear is fixedly connected with the first pulley and is coaxial with the first pulley; the first gear cooperates with the engaging teeth;
the first pulley is provided with a first annular groove and a second annular groove, wherein one end of the control member is fixed in the first annular groove, and the other end of the control member is fixed in the second annular groove and is wound in the second annular groove.

4. The endoscopic needle driving assembly according to claim 2, wherein
the driving assembly comprises two second gears cooperating with the engaging teeth, and an annular groove is provided on each of the two second gears;
one end of the control member is fixed in the annular groove of one of the two second gears, and the other end of the control member is fixed and wound in the annular groove of the other one of the two second gears.

5. The endoscopic needle driving assembly according to claim 2, wherein
the rotating body is a bevel gear.

6. The endoscopic needle driving assembly according to claim 1, wherein
the driving assembly comprises a pulley, and the control member is redirected by the pulley and then connected to the rotating body.

7. The endoscopic needle driving assembly according to claim 6, wherein
the rotating body is the second pulley, and the second pulley is provided with a third annular groove and a fourth annular groove;
two pulleys are provided;
one end of the control member is fixed in the third annular groove after being redirected by one pulley; and the other end of the control member is fixed in the fourth annular groove after being redirected by another pulley, and is wound in the fourth annular groove.

8. The endoscopic needle driving assembly according to claim 1, wherein
the rotating body is provided with an arc-shaped sliding groove; the arc-shaped sliding groove is wound around the rotating body; and in an axial direction of the rotating body, the arc-shaped sliding groove extends from one end of the rotating body to the other end of the rotating body;
the driving assembly comprises a sliding block, wherein the sliding block is connected with the control member and is slidably mounted in the arc-shaped sliding groove.

9. The endoscopic needle driving assembly according to claim 8, wherein
the driving assembly further comprises a fixing shell, wherein the fixing shell is fixed in the housing assembly, and along a length direction of the fixing shell, the fixing shell is provided with a sliding hole; a portion of the sliding block is located in the fixing shell and is slidable in the fixing shell, and another portion of the sliding block passes through the sliding hole and is inserted into the arc-shaped sliding groove; and
a spring, wherein the spring is arranged in the fixing shell and is compressed in place between the sliding block and the fixing shell.

10. The endoscopic needle driving assembly according to any one of claims 1 to 9, wherein
the housing assembly comprises: a mounting shell; and
a fixing tube, wherein the fixing tube is fixed in the mounting shell, and the rotating body is sleeved over the fixing tube and is able to rotate relative to the fixing tube.

11. The endoscopic needle driving assembly according to claim 10, wherein
the fixing tube comprises a fixing section and a mounting section, a diameter of the mounting section is smaller than a diameter of the fixing section, and the rotating body is fixed on the mounting section.

12. The endoscopic needle driving assembly according to claim 10, wherein
the fixing tube comprises a tube body and a transfer shaft, the rotating body is able to be mounted on the transfer shaft in a rotatable manner, and a projection of the rotating body on the tube body at most covers a part of the through hole of the tube body.

13. The endoscopic needle driving assembly according to any one of claims 1 to 9, wherein
a side wall of the suturing groove is provided with a cut surrounding the suturing groove, and the cut communicates with the suturing hole.

14. The endoscopic needle driving assembly according to any one of claims 1 to 9, wherein
the rotating body is coaxial with the arc-shaped suturing needle.

15. The endoscopic needle driving assembly according to any one of claims 1 to 9, wherein
a rotation axis of the rotating body has an angle with a rotation axis of the arc-shaped suturing needle.

16. A suturing device,
comprising a suture, an endoscope, and the endoscopic needle driving assembly according to any one of claims 1 to 15, wherein the suture is connected with the arc-shaped suturing needle of the endoscopic needle driving assembly, and the endoscope is sleeved and connected with the fixing tube of the endoscopic needle driving assembly.
